# EUROPEAN PATENT APPLICATION

(11) **EP 1 350 536 A1**
(43) Date of publication of application: **08.10.2003**
(21) Application number: 02425132.4
(22) Date of filing: 07.03.2002
(51) Int. Cl.: A61M 25/06

(54) **Safety butterfly needle for venipuncture with needle cover device**

(71) Applicant: Restelli, Sergio, 00100 Rome (IT); Righi, Nardino, 20047 Brugherio (Milano) (IT); Rossi, Roberto, 20151 Milano (IT)
(72) Inventor: Restelli, Sergio, 00100 Rome (IT); Righi, Nardino, 20047 Brugherio (Milano) (IT); Rossi, Roberto, 20151 Milano (IT)
(74) Representative: Petruzziello, Aldo

(57) **Abstract**

A butterfly needle (100) for venipuncture comprises a body (1) with two outwardly protruding flexible wings (2) foldable by manual gripping by the user from a diverging position to a converging position, and a needle (9) mounted axially in the fore end (32) of a needle-carrier (3) which has a tail (8) protruding rearward from said body (1) to couple with the connection of a flexible tube designed to be connected to a syringe or bottle. A substantially cylindrical needle cover (5), hollow on the inside, is mounted to slide axially inside the body (1) to pass from a retracted position of use in which it leaves the needle (9) free and protruding forward from the body (1) to a forward position of safety in which it protrudes forward from the body (1) to cover the needle (9).

## Description

The present invention refers to a safety butterfly needle for venipuncture provided with a needle cover device.

As is known, butterfly needles for venipuncture are widely available on the market. Said butterfly needles are generally used for intravenous injections, infusions, blood sampling, transfusions and the like. Although specific reference will be made herein to injections, it is to be understood that the butterfly needle according to the invention can also be used for blood sampling and the like.

A butterfly needle generally comprises a needle proper to penetrate into the vein. Said needle is supported integrally by a needle-carrier on which two flexible tongues shaped like butterfly wings are mounted.

The rear end of the needle is connected to a small flexible plastic tube. The tube ends in a wider mouth, which is closed by a stopper. The mouth of the tube is designed to couple with the fore end of a syringe filled with a solution to be injected for intravenous injections or with the head of a container or bottle for intravenous infusions.

Said flexible tongues perform a dual function: that is to say, they act as a grip for the operator who must perform the injection and they act as a fixing means for fixing the needle to the patient's skin to secure it in place during the injection. Once the treatment has been completed, the user folds the tongues to make them converge and removes the needle from the vein.

The assembly of butterfly needle, tube, syringe, bottle etc. cannot be re-used and therefore is sent for disposal. This operation proves extremely dangerous because the butterfly needle remains exposed and this leads to the risk of accidental needle sticks both by the operator responsible for the injection and by the personnel responsible for disposal.

The object of the present invention is to overcome the drawbacks of the prior art by providing a safety butterfly needle with a needle-cover device that is able to avoid accidental needle sticks.

Another object of the present invention is to provide such a safety butterfly needle that is practical and simple for the user to use.

Yet another object of the present invention is to provide such a safety butterfly needle that is economical and simple to make.

These objects are achieved in accordance with the invention with the characteristics listed in appended independent claim 1.

Advantageous embodiments of the invention are apparent from the dependent claims.

The safety butterfly needle for venipuncture according to the invention comprises a substantially cylindrical body with two outwardly protruding flexible tongues which can be manually gripped by the user and bent from a diverging to a converging position.

A needle is axially mounted in the head of a needle-carrier coupled to the body, so that the needle protrudes axially forward from the body. The needle carrier has a tail protruding rearward from the body to couple with the connection of a flexible tube destined to be connected to a syringe or to a bottle for injection of a solution or for collection of blood.

The main characteristic of the invention is that said butterfly needle has a substantially cylindrical needle-cover device, hollow on the inside, mounted so that it can slide axially inside the body of the butterfly needle. In this manner the needle-cover device can pass from a retracted position of use in which it leaves the needle free and protruding forward from the body to a forward position of safety in which the needle-cover device protrudes forward from the body to cover the needle.

The advantages of the butterfly needle with needle-cover device according to the invention are obvious. In fact, once the injection has been performed the needle is protected by the needle-cover device in a safe position, thus avoiding accidental injuries.

Further characteristics of the invention will be made clearer by the detailed description that follows, referring to a purely exemplary and therefore non-limiting embodiment thereof, illustrated in the appended drawings, in which:
Figure 1 is an exploded perspective view illustrating a butterfly needle according to the invention, with a needle-cover device and a needle cap;
Figure 2 is a top plan view of the butterfly needle of Figure 1 assembled and in the position of use, in which the needle cap is not shown;
Figure 3 is a plan view from the rear of the assembled butterfly needle, taken from the right-hand side of Figure 2;
Figure 4 is an axial sectional view of the butterfly needle in the position of use, taken along the plane of section IV-IV of Figure 2;
Figure 5 is a top plan view of the butterfly needle according to the invention, shown in the safety position;
Figure 6 is an axial sectional view of the butterfly needle in the safety position, taken along the plane of section VI-VI of Figure 5.

The butterfly needle according to the invention, denoted as whole with reference numeral 100, is described with the aid of the figures.

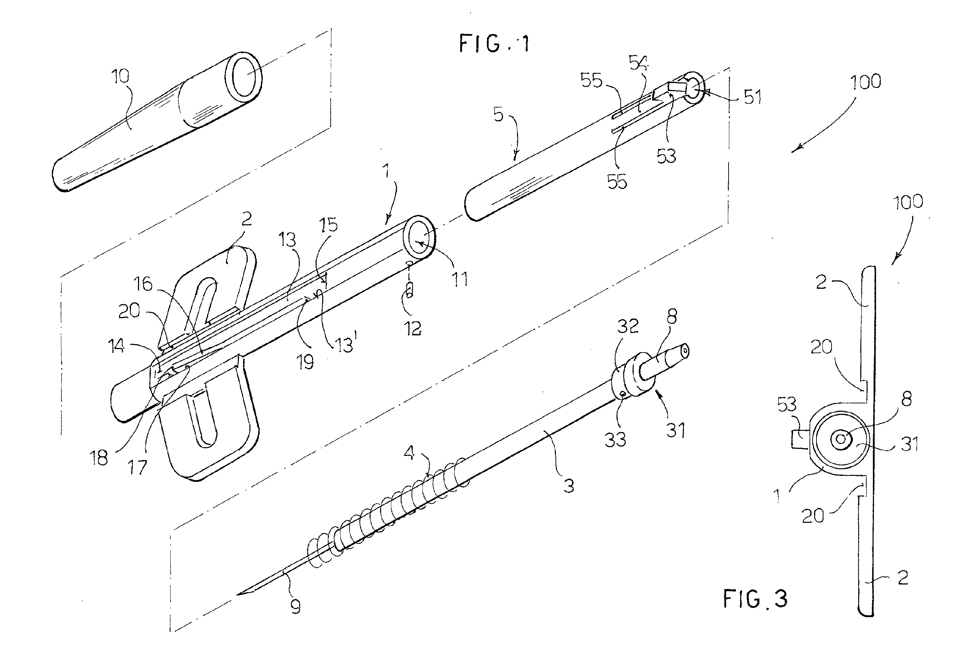

With reference for now to Figure 1, the butterfly needle 100 comprises a main body 1, substantially cylindrical in shape, on which two flexible tongues 2 are mounted. The tongues 2 are mounted in the front part of the body 1 and protrude in opposite directions with respect to the axis of the body. Near the body 1 the tongues 2 have longitudinal grooves 20, which act as a fold line to allow the tongues 2 to be folded along the grooves 20. In this manner the tongues 2, bending along the fold line 20, can pass from an open position, shown in the figures, to a closed position, in which they converge, drawing nearer to each other so that they can be grasped by the user.

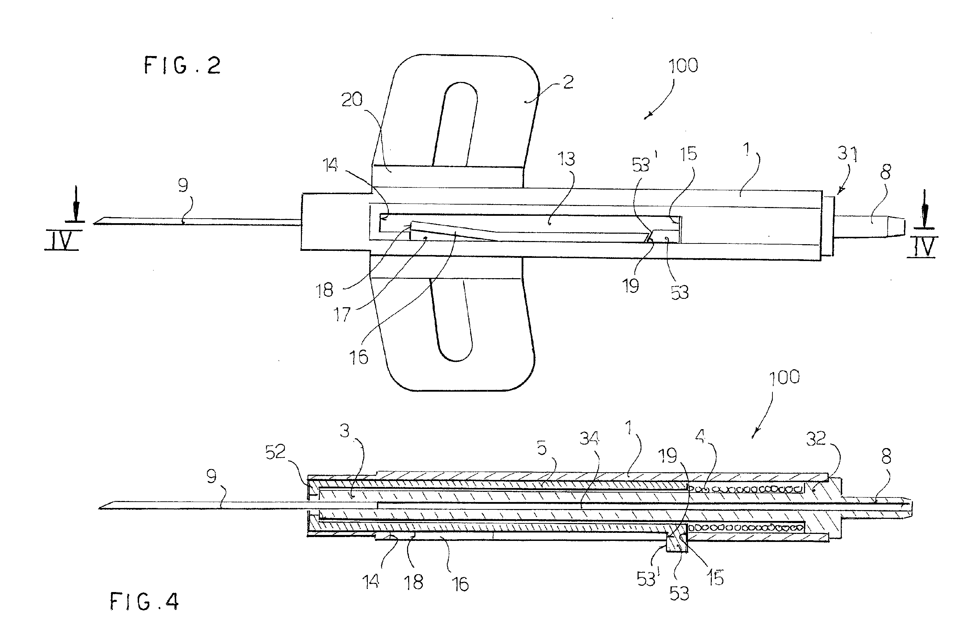

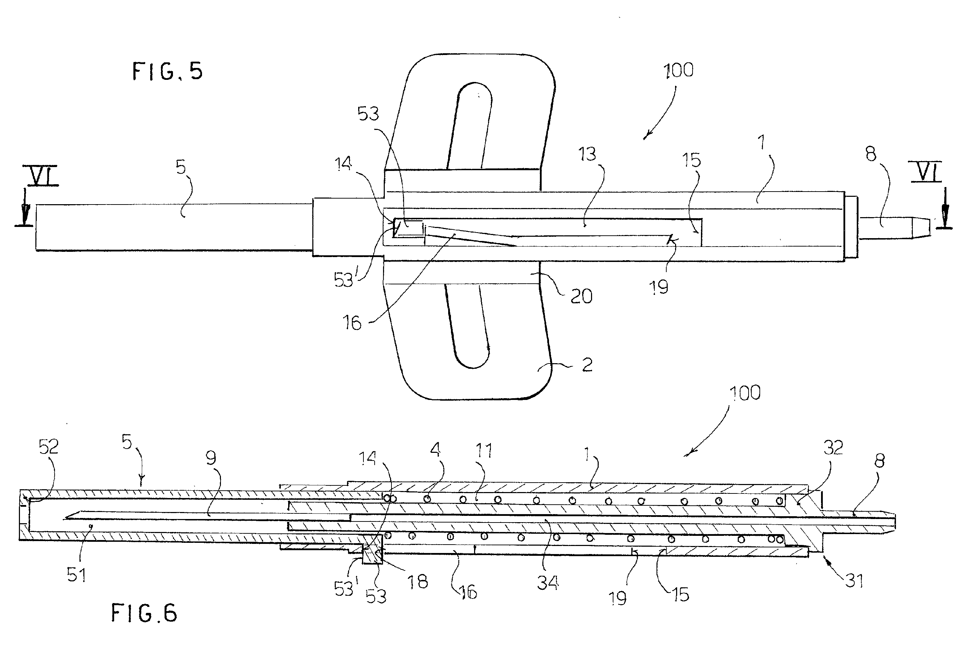

A needle 9 is axially mounted in the fore end of a needle-carrier 3. As shown in Figures 4 and 6, the needle carrier 3 has an axial channel 34 open at the front and rear for passage of the liquid to be injected. The needle carrier 3 must be mounted inside the body 1 so that the needle 9 protrudes forward from the body 1 to perform the injection. The needle 9 is covered by a substantially frusto conical needle cap 10 that snap engages in the fore end of the body 1.

The needle-carrier 3 has a tail 8 that protrudes rearward from the body 1 to couple with a special connection of a flexible tube (not shown in the figures) intended to be connected to a syringe or to a bottle containing the solution to be injected or intended to receive the blood withdrawn. In the rear part the needle carrier 3 has a discoid flange 31 able to abut against the rear edge of the body 1 and a cylindrical tang 32 with a larger diameter than the body of the needle-carrier able to engage in the rear part of the body 1. The cylindrical tang 32 defines an annular abutment surface around the body of the needle carrier 3.

A radial hole 33 is formed in the tang 32 of the needle carrier to receive a securing pin 12 that protrudes radially inward in the rear part of the body 1, so as to firmly secure the needle-carrier 3 inside the body 1, preventing axial movements and rotations of the needle-carrier 3 with respect to the body 1.

The body 1 is substantially cylindrical in shape and hollow on the inside, having a cylindrical chamber 11 open at the front and rear. The body 1 has a longitudinal slot 13 in its side surface. The longitudinal slot 13 is disposed at an angular distance of about 90° with respect to the wings 2. The longitudinal slot 13 is delimited by a front abutment surface 14 and a rear abutment surface 15.

An elastic tongue 16 that has a part protruding toward the axis of the slot 13 is provided in a side wall of the slot 13. The protruding part of the elastic tongue 16 is separated from the side wall of the slot 13 by means of a cut 17. In this manner the tongue 16 can bend radially moving away from or toward the axis of the longitudinal slot 13.

The flexible tongue 16 has an abutment surface 18 level with its free front end, which is opposed to the front abutment surface 14 of the slot 13.

In the rear part of the slot 13 a lateral recess 13' forming an inclined abutment surface 19 that is opposed to the rear abutment surface 15 of the slot 13 is provided. In practice the slot 13 is substantially L-shaped at its rear to allow a bayonet joint.

The butterfly needle 100 comprises a needle-cover device 5 in the form of a cylindrical sleeve having a slightly greater length than that of the needle 9. The needle-cover device 5 is hollow on the inside and has an axial chamber 51 open at the front and rear.

The outside diameter of the needle-carrier device 5 is slightly smaller than the inside diameter of the body 1, so as to be able to slide therein, and the inside diameter of the needle-cover device 5 is slightly greater than the outside diameter of the needle-carrier 3. In this manner, the needle-carrier 3 can be mounted inside the needle-cover device 5 and the needle-cover device 5 can be mounted inside the body 1.

As shown in particular in Figures 4 and 6, the needle-cover device 5 has at its front a collar 52 that protrudes inward so as to define a radial abutment surface.

Returning to Figure 1, in the rear part of the needle-cover device 5 a prong 53 that protrudes radially outward from the body of the needle-cover device 5 has a chamfered front surface 53', with the same inclination as said abutment surface 19 formed in the body 1. The prong 53 is mounted on a flexible tongue 54 formed longitudinally on the body of the needle-cover device 5.

The tongue 54 is obtained by means of two parallel longitudinal slots 55 formed on the body of the needle-cover device 5. In this manner the tongue 54 can bend radially towards the inside of the body of the needle-cover device 5.

Assembly and operation of the butterfly needle 100 will now be described.

The needle-cover device 5 is inserted inside the body 1 making the tongue 54 bearing the prong 53 bend radially inward until the prong 53 enters the slot 13 of the body 1 causing elastic return of the tongue 54. To be precise, the prong 53 is disposed between the rear abutment surface 15 of the slot 13 and the inclined abutment surface 19 of the body 1. In this manner axial movements of the needle-cover device 5 are avoided.

A helicoidal spring 4 and the needle-carrier 3 are then inserted respectively into the body 1 from the rear. When the front end of the needle-carrier 3 abuts against he collar 52 of the needle-cover device 5, the needle 9 protrudes forward out of the body 1 and the spring 4 is compressed inside the rear part of the body 1 with its ends in abutment respectively against the rear edge of the needle-cover device 5 and the radial abutment surface generated by the tang 32 of the needle-carrier. In this situation the locking pin 12 is inserted and engages inside the radial hole 33 of the tang 32 of the needle-carrier, blocking he needle-carrier 3.

Of course, the above operations can be inverted, in that the needle-cover device 5 can be mounted after assembly of the needle-carrier 3 and the spring 4. In any case, the needle-cover device 5 remains locked in position through the action of the spring 4, which biases the inclined surfaces 19 and 53' into mutual contact.

Once the injection has been performed, the user pulls the prong 53 backward with a fingertip to overcome the force of the spring 4, and rotates it slightly, so as to free the inclined surfaces 19 and 53' from each other. Consequently, the prong 53 is disposed axially in the longitudinal slot 13 and the spring 4 releases causing an axial forward movement of the needle-cover device 5.

During the axial forward movement of the needle-cover device 5, the prong 53 of the needle-cover device slides, guided in the longitudinal seat 13 of the body, causing bending of the elastic tongue 16 of the body, until the prong 53 abuts against the front abutment surface 14 of the longitudinal slot 13 of the body.

In this situation shown in Figures 5 and 6, the needle-cover device 5 is in its forward safety position and the needle 9 is protected inside the chamber 51 of the needle-cover device. It should be noted that, in this situation, the elastic tongue 16 of the body returns elastically so that its free abutment surface 18 abuts against the prong 53. Consequently the prong 53 is comprised between the front abutment surface 14 of the longitudinal slot and the abutment surface 18 of the elastic tongue 16 and thus any axial movement of the needle cover device 5 is prevented.

Numerous changes and modifications of detail within the reach of a person skilled in the art can be made to the present invention without departing from the scope of the invention as set forth in the appended claims.

## Claims

1. A butterfly needle (100) for venipuncture comprising
- a substantially cylindrical body (1) with two outwardly extending flexible tongues (2) that can be manually grasped and bent by the user from a diverging position to a converging position, and
- a needle (9) mounted axially in the fore end (32) of a needle-carrier (3) coupled to said body (1) so that the needle (9) protrudes forward from said body (1), said needle-carrier providing a tail (8) protruding rearward from said body (1) to couple with the connection of a flexible tube intended to be connected to a syringe or bottle,
**characterized in that** it comprises
a needle-cover device (5), substantially cylindrical in shape and hollow on the inside, mounted to slide axially inside the body (1) to pass from a retracted position of use in which it leaves the needle (9) free, protruding forward from the body (1) to a forward position of safety in which it protrudes forward from the body (1) to cover the needle (9).

2. A butterfly needle (100) according to claim 1, **characterized in that** spring means (4) are disposed inside said body (1), between the rear end of said needle-cover device (5) and an abutment surface (32) of said needle-carrier (3) or of said body (1), said spring means (4) being loaded when said needle-cover device (5) is in the retracted position of use.

3. A butterfly needle (100) according to claim 2, **characterized in that** said needle-carrier (3) comprises at the rear a cylindrical tang (32) with a larger diameter than the body of the needle-carrier, having a radial hole (33) to receive a securing pin (12) to secure the needle-carrier (3) to said body (1), said cylindrical tang (32) of the needle-carrier (3) defining an annular abutment surface against which one end of said spring means (4) abuts.

4. A butterfly needle (100) according to any one of the preceding claims,
**characterized in that** it comprises end of stroke and locking means able to lock said needle-cover device (5) in said retracted position of use and in said forward position of safety.

5. A butterfly needle (100) according to claim 4, **characterized in that** said body (1) comprises a longitudinal slot (13), defined by a front abutment surface (14) and a rear abutment surface (15) and said needle-cover device (5) comprises a prong (53) protruding radially therefrom to engage in said longitudinal slot (13), so that said prong (53) protrudes outward from the body (1) to be able to be operated manually by the user, said rear (15) and front (14) abutment surfaces of the slot (13) forming the end-of-stroke means to stop the backward and forward stroke of said needle-cover device (5).

6. A butterfly needle (100) according to claim 5, **characterized in that** said longitudinal slot (13) has, in its rear part, a lateral recess (13') forming an inclined abutment surface (19) that is opposed to said rear abutment surface (15) and **in that** said prong (53) of the needle cover device (5) has a chamfered front surface (53') with the same inclination as said abutment surface (19), so that, when the needle cover device is in the retracted position of use, it remains locked in position by the action of the spring (4) which biases the aforementioned inclined surfaces (19) and (53') into mutual contact.

7. A butterfly needle (100) according to claim 5 or 6, **characterized in that** in said longitudinal slot (13) a flexible tongue (16) is provided, having an abutment surface (18) opposed to the front abutment surface (15) of the slot (13) so that, when the needle cover device is in the forward safety position, the prong (53) of the needle-cover device is retained between said abutment surface (18) of the tongue (16) and the front abutment surface (14) of the longitudinal slot (13).

8. A butterfly needle (100) according to claim 7, **characterized in that** said flexible tongue (16) of said body (1) is formed by means of a longitudinal cut (17) on said body (1).

9. A butterfly needle (100) according to any one of claims 5 to 8, **characterized in that** said prong (53) is mounted on a flexible longitudinal tongue (54) of said needle-cover device (5).

10. A butterfly needle (100) according to claim 9, **characterized in that** said flexible longitudinal tongue (54) of said needle cover device (5) is formed by means of parallel longitudinal cuts (55) on said needle cover device (5).
